# EUROPEAN PATENT APPLICATION

(11) **EP 2 193 812 A1**
(43) Date of publication of application: **09.06.2010**
(21) Application number: 10157988.6
(22) Date of filing: 17.09.2004
(51) Int. Cl.: A61L 27/18, A61L 24/04

(54) **Use of a curable elastomer-precursor for a medical treatment**

(30) Priority: 19.09.2003 US 504349 P; 15.03.2004 US 553100 P; 08.04.2004 US 560393 P
(62) Divisional of application: 04077581.9
(71) Applicant: Broockeville Corporation N.V., Willemstad, Curaçao (AN)
(72) Inventor: De Vries, Jan Albert, 7021 HH, ZELHEM (NL)
(74) Representative: Volmer, Johannes Cornelis

(57) **Abstract**

Disorders of the vertebrae of a living creature, for example derived from osteoporosis, can be treated by injection of an elastic form stable material, preferably a curable elastomer precursor composition into the respective vertebral body. The invention relates to the use of a curable elastomer precursor composition in the manufacture of a bone cement composition for treating a disordered vertebral body in a living creature, in particular a human being

## Description

The present invention relates to the manufacture of a composition for treatment of a disordered vertebral body in a living creature, in particular a human being, by vertebroplasty. More specifically this aspect relates to the use of a certain composition suitable for use in such a manufacturing method.

Percutaneous vertebroplasty comprises the injection of bone cement into a vertebral body to be treated via a percutaneous route, usually under X-ray guidance, such as lateral projection fluoroscopy. The bone cement is injected as a paste or semi-liquid from a suitable cement gun or injection system via a needle, that has been passed into the vertebral body. Vertebroplasty is usually applied to patients that suffer from osteoporotic fractures, malignant metastatic disease and benign tumors of the bone. The bone cement once injected should provide a reinforcement to and improved compressive strength of the vertebral body. In addition to these reinforcing and strengthening properties it is desirable that the starting bone cement composition is of a viscosity that allows it to flow into the fracture planes.

At present almost all bone cement is based on polymethyl methacrylate. This type of bone cement is prepared from a mixture of methyl methacrylate powder and a suitable radiopaque agent (opacifier) to which a liquid monomer is added. Then the resulting cement should be injected within a limited period of time, e.g. 5 to 10 minutes, before the cement starts to thicken and becomes unworkable. While the cement is being injected, continuous X-ray imaging is performed in order to watch carefully whether the cement is deposited in the appropriate position and proportion, typically some cubic centimetres, within the vertebral body. The cement thus injected is then cured within the vertebral body to a hard material.

However, it has appeared that this type of bone cement gives some disadvantages. Due to its hardness and strength the cured material can easily distort the brittle bone material of the vertebral body into which it has been injected. Furthermore there is a risk of leakage of material from the vertebral body, thereby cutting through tissue and nerves.

Thus there is a need for a vertebroplasty material, which does not present the above disadvantages, or at least to a lesser extent.

The present invention provides for the use of a curable elastomer-precursor composition in the manufacture of a bone cement composition for treating a disordered vertebral body in a living creature, in particular a human being. This composition is to be used in a vertebroplasty method of treating a disordered vertebral body in a living creature, in particular a human being, the method comprising the step of injecting a curable bone cement composition in said vertebral body, wherein said curable bone cement comprises a curable elastomer-precursor composition. After curing, an elastic form stable material is obtained.

According to the invention, a creature such as a human being in need of a vertebroplasty treatment e.g. derived from osteoporosis, is treated by reinforcing and strengthening the respective vertebral body or bodies. To this purpose a bone cement is injected into the selected vertebral body, wherein the bone cement is a curable elastomer-precursor composition, resulting into an elastic form stable material. Due to its properties with respect to elasticity and dimensional stability the forces exerted on the brittle vertebral body upon and after injecting the bone cement composition are smaller compared to the state of the art, thereby reducing the risk of breaking or disrupting the body thus treated. After curing this material being non-resorbable, is a solid but still flexible mass that cannot migrate through the body, but holds its initial position where it is applied.

Typically the vertebroplasty method using the bone cement composition according to the invention comprises the following steps. The patient is examined usually by CT and/or MR imaging in order to provide the data upon which a decision about the vertebral body to be treated is made. The patient to be treated is positioned in a prone position, and the skin covering the vertebral body is prepared. The patient is anaesthetized, usually locally by injecting a suitable anaesthetic into the skin underlying fat and into the periosteum of the pedicle to be entered. Next a skin incision is made using a scalpel or other surgical instrument. A needle having a suitable gauge is selected and passed down the pedicle into the respective vertebral body. If desired, any unwanted leakage can be detected by injecting a suitable X-ray contrast liquid into the vertebral body and performing a vertebrogram. Meanwhile the bone cement is prepared and is then injected, while monitoring the position of the needle, the position and amount of injected cement, e.g. by lateral X-ray projection fluoroscopy imaging. For that purpose a suitable opacifier is added to the bone cement. The injection is stopped when the filling of the vertebral body has been completed to the desired extent. If the cement starts to flow into unwanted locations, injection will also be interrupted.

Preferably the elastic form stable material comprises an elastomer-precursor composition which is cured in situ. That is to say, the elastomer precursor is prepared in advance and then applied to the appropriate vertebral body by suitable equipment.

The invention also relates to a method of preparing a composition for treating a disordered vertebral body in a living creature, in particular a human being, comprising an elastic form stable material. Usually this manufacturing method comprises the steps of providing the starting materials of the elastic form stable materials in the appropriate ratio into separate containers (or separated chambers that may be present in one mixing/dispensing device), and packaging thereof.

Preferred embodiments of the invention are defined in the subclaims.

According to a preferred embodiment, the vertebroplasty composition comprises a curable elastomer-precursor composition. Commercially available medical grade silicone elastomers are preferred materials for use as polymer precursor in this composition. A more preferred material is poly(dimethyl siloxane) such as hydroxyl-end-blocked poly(dimethyl siloxane). Such silicone elastomers of medical grade as pourable, two-component silicone are available from e.g. NuSil Technology. These silicone elastomers are fast curing materials. For these types of elastomers propyl orthosilicate is a useful cross-linking agent. Fillers and diluents (medicinal fluids such as known under the trade name Dimeticonum) in order to reduce viscosity may be added as needed. It is preferred that a well flowable composition is used, so that even small hair cracks are filled with the composition. An initiator like tin (II) octoate initiates the polymerisation reaction with splitting of propanol. The reaction proceeds without the generation of sensible heat, which is also benificial to the patient treated. Silphenylene polymer can be used in a similar way. In order to be able to trace by X-ray monitoring the position of the curing/cured applied composition, in a preferred embodiment the composition comprises a radiopaque material, such as silver powder, barium sulfate or bismuth trioxide. Silver powder is the most preferred radiopaque material, because compared to the other examples a smaller proportion is needed in the composition.

Here it is noted that a composition as explained above is known per se as a material suitable for the non-surgical, reversible sterilization of females. In this known sterilization method the composition is injected in the oviduct portion adjacent the uterus, where it forms in situ a block or plug in the oviduct, thereby preventing the passage of ovum from the ovaries to the uterus and sperm from entering the oviduct and thus conception. See e.g. US-A-4,245,623.

A preferred bone cement composition comprises about 60 -75 % by weight poly(dimethyl siloxane), about 2-5 % cross-linking agent, a diluent in the range of 10-20 % and about 10-20 % radiopaque powder, based upon the weight of the entire composition. An even more preferred composition comprises about 68 wt.% poly(dimethyl siloxane), about 4 % cross-linking agent, about 13 % dimeticonum and about 16 % silver powder.

In the vertebroplasty method using the composition according to the invention the composition is advantageously prepared in advance in a mixing-dispensing device. Such a device, wherein the function of mixing the components is combined with the function of dispensing the thus prepared mixture, is known per se, e.g. from the above US patent, the content of which is incorporated in its entirety by reference.

As already indicated above, the invention also relates to a method of preparing an injection composition for injection into a disordered vertebral body of a living creature, in particular a human being, said composition comprising an elastic form stable material.

The above-mentioned preferred features mentioned above are similarly applicable to the preparation method according to the invention.

Advantageously the injection composition is packaged as a kit of parts, comprising at least a first container filled with an elastomer precursor and optionally a diluent, and a second container filled with a cross-linking agent for this elastomer precursor. More preferably, the composition is packaged in a mixing-dispensing device, comprising such containers and a temporarily seal between the containers, wherein one of these containers is provided with a stirrer which can be operated manually or powered by an external source. An example of such a device is also known from the above-mentioned US patent. Devices of this type can be used for injecting the thus prepared precursor composition by connecting a suitable flexible tube to the container acting as a mixing chamber and providing an appropriate needle at the other end of the tube.

In the invention a flowable composition is prepared from the various components, preferably in a combined mixing-dispensing device as explained above, and then immediately used. In order to more accurately position the deposit of the composition, preferably the needle, in addition to an open tip, has one or more exits at the side face directly adjacent to the tip.

Use of this modified needle allows positioning the composition more easily at the required positions. Thereafter the needle is retracted and the composition is allowed to cure in situ. If necessary, the above actions can be repeated as needed.

## Claims

1. Use of a curable elastomer-precursor composition in the manufacture of a bone cement composition for treating a disordered vertebral body in a living creature, in particular a human being.

2. Use according to claim 1, wherein the curable elastomer-precursor composition comprises a silicone elastomer.

3. Use according to claim 2, wherein said silicone elastomer is poly(dimethoxy siloxane).

4. Use according to one of the preceding claims 1-3, wherein said composition comprises a silicone elastomer, a cross-linking agent and a diluent.

5. Use according to one of the preceding claims 1-4, wherein said composition comprises a radiopaque material.

6. Use according to one of the preceding claims 1-5, wherein said composition is packaged as a kit of parts, comprising at least a first container with said silicone elastomer, and a second container with said cross-linking agent.

7. Use according to claim 6, wherein said kit of parts is a mixing-dispensing device, comprising said containers and a temporarily sealing between the containers, wherein one container is provided with a movable stirrer.

8. Use of an elastic form stable material in the manufacture of a composition for treatment of a disordered vertebral body in a living creature, in particular a human being.
